(19) Europäisches Patentamt
European Patent Office
Office européen des brevets

(11) Veröffentlichungsnummer: **0 053 756**
**B1**

(12) # EUROPÄISCHE PATENTSCHRIFT

(45) Veröffentlichungstag der Patentschrift: **12.09.84**

(51) Int. Cl.³: **C 07 D 309/18**, A 01 N 43/16

(21) Anmeldenummer: **81109863.1**

(22) Anmeldetag: **25.11.81**

(54) **2,6-Diethyl-3,5-dimethyl-2,3-dihydro-4H-pyran, Verfahren zu seiner Herstellung und seine Verwendung zur Früherkennung, Lokalisierung und Bekämpfung von Lasioderma serricorne F.**

(30) Priorität: **05.12.80 DE 3045909**

(43) Veröffentlichungstag der Anmeldung: **16.06.82 Patentblatt 82/24**

(45) Bekanntmachung des Hinweises auf die Patenterteilung: **12.09.84 Patentblatt 84/37**

(84) Benannte Vertragsstaaten: **AT BE CH DE FR LI LU NL SE**

(56) Entgegenhaltungen:
**US - A - 2 858 322**

**BULLETIN DE LA SOCIETE CHIMIQUE DE FRANCE, 1978, Nr. 3-4, II, Paris(FR) R. SEMET et al.: "Monoréduction de méthyl dihydro-3,4 pyrones-2 delta-lactones d'énol) par des ions hydrures", Seiten 185-192**
**BULLETIN DE LA SOCIETE CHIMIQUE DE FRANCE, 1969, No. 11, PARIS (FR) G. DESCOTES et al.: "Cyclodéshydratation de tétrahydrofuryl carbinols IV. - Cas des tétrahydrofuryl carbinols tertiaires", Seiten 4151-4154**
**BULLETIN DE LA SOCIETE CHIMIQUE DE FRANCE, Oktober 1967, No. 10, PARIS (FR) J. COLONGES et al.: "Contribution à l'étude des nitriles delta-cétoniques", Seiten 3881-3888**

(73) Patentinhaber: **B.A.T. Cigaretten-Fabriken GmbH, Alsterufer 4, D-2000 Hamburg 36 (DE)**

(72) Erfinder: **Levinson, Hermann, Prof. Dr., Keltenweg 7, D-8131 Starnberg-Perchting (DE)**
Erfinder: **Levinson, Anne-Rose, Dr., Keltenweg 7, D-8131 Starnberg-Perchting (DE)**
Erfinder: **Francke, Wittko, Dr. Dipl.-Chem., Am Vorwerksbusch 5, D-2057 Reinbek (DE)**
Erfinder: **Volker, Heemann, Dr., Klosterbergenstrasse 24, D-2057 Reinbek (DE)**

(74) Vertreter: **Glawe, Delfs, Moll & Partner Patentanwälte, Rothenbaumchaussee 58 Postfach 2570, D-2000 Hamburg 13 (DE)**

(56) Entgegenhaltungen: (Fortsetzung)
**BULLETIN DE LA SOCIETE CHIMIQUE DE FRANCE, 1967, No. 7, PARIS (FR) G. DESCOTES et al.: "Déshydratation catalytique de tétrahydrofuryl-carbinols substitués. II-Etude du mécanisme de l'agrandissement du cycle", Seiten 2472-2477**
**CHEMICAL ABSTRACTS, Band 79, 1973, Seite 411, Zusammenfassung, 91889s, COLUMBUS, OHIO (US)**
**CHEMICAL ABSTRACTS, Band 79, 1973, Seite 449, Zusammenfassung, 31959e, COLUMBUS, OHIO (US)**
**CHEMICAL ABSTRACTS, Band 92, 1980 Seite 519-520, Zusammenfassung, 145979k, COLUMBUS, OHIO (US)**
**NATURWISSENSCHAFTEN, 60. Jahrgang, Heft 3, März 1981, BERLIN (DE) H.Z. LEVINSON et al.: "The Pheromine Activity of Anhydroserricornin and Serricornin for Male Cigarette Beetles" (Lasioderma serricorne F.) Seiten 148-149**

EP 0 053 756 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft 2,6-Diethyl-3,5-dimethyl--2,3-dihydro-4H-pyran der Formel

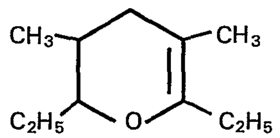

Die Erfindung betrifft weiterhin Verfahren zur Herstellung der vorgenannten Verbindung, Mittel zur Früherkennung, Lokalisierung und Bekämpfung von Lasioderma serricorne F., die die genannte Verbindung sowie übliche feste oder flüssige Trägerstoffe, Insektizide und/oder Insektistatica enthalten sowie die Verwendung der vorgenannten Verbindung zur Früherkennung, Lokalisierung und Bekämpfung von Lasioderma serricorne F.

Die Erfindung betrifft weiterhin die optischen Isomeren der eingangs genannten Verbindung.

Es ist bekannt, dass 4,6-Dimethyl-7-hydroxy-nonan-3-on (Serricornin) eine Pheromonkomponente des weiblichen Tabakkäfers Lasioderma serricorne F. ist, vgl. Tetrahedron Letters *25*, 2361/1979. Eine vielstufige Synthese zur Herstellung von Serricornin ist in Agric. Biol. Chem., 44 (9), 2259 bis 2260 (1980), beschrieben.

Die vorliegende Erfindung beruht auf der Erkenntnis, dass 2,6-Diethyl-3,4-dimethyl-2,3-dihydroxy--4H-pyran (Anhydroserricornin) eine weitere, bisher nicht bekannte Pheromonkomponente des weiblichen Tabakkäfers Lasioderma serricorne ist. Die Konzentration von Anhydroserricornin im Sexuallockstoff des Tabakkäfers ist etwa 10mal geringer als die des Serricornins; überraschenderweise wurde jedoch auch gefunden, dass Anhydroserricornin bezüglich seiner Lock- und Paarungswirkung etwa 1000mal stärker als Serricornin ist.

Die Verbindung der Erfindung kann erhalten werden, indem man 4,6-Dimethyl-7-hydroxy-3-nonanon cyclisiert.

Die Erfindung ist weiterhin auf die Verwendung von Anhydroserricornin zur Früherkennung, Lokalisierung und Bekämpfung von Lasioderma serricorne F. gerichtet. Hierzu kann die Verbindung der Erfindung, die einen synthetisch hergestellten Lockstoff darstellt, in dem befallenen Gebiet, den Lagerräumen oder dergleichen ausgebracht werden, z.B. als Köderfallen mit üblichen festen oder flüssigen Trägerstoffen, Insektiziden und/oder Insektistatica. Geeignete Trägermaterialien sind z.B. Papier, Wellpappe, Kunststoffe, Gummi oder dergleichen. Auf diese Weise kann bereits ein geringfügiger Befall mit Tabakkäfern festgestellt werden, so dass rechtzeitig geeignete Massnahmen zu deren Bekämpfung ergriffen werden können. Auch die direkte Bekämpfung ist möglich, z.B. durch Überdosierung des Sexuallockstoffes oder Kombination desselben mit Insektiziden oder Insektistatica.

Im folgenden wird die Herstellung von Serricornin (Ausgangsprodukt; Stufen A bis D) sowie von Anhydroserricornin (Verbindung der Erfindung; Stufe E) beschrieben.

A. N-(1-Ethyl-1-propenyl)-pyrrolidin

Zu einer Mischung von 86,15 g Pentanon-3 (1 Mol), 213,36 g Pyrrolidin (3 Mol) und 1,8 l Pentan wurden unter trockenem Stickstoff eine Lösung von 104,35 g Titantetrachlorid (0,55 Mol) in 100 ml Pentan zugetropft. Die Temperatur des Reaktionsgemisches wurde dabei durch Eiskühlung zwischen 0°C und 10°C gehalten. Anschliessend wurde noch einige Stunden bei Raumtemperatur gerührt und über Nacht stehen gelassen. Das Pentan wurde vom Niederschlag abdekantiert und dieser mit Eis vorsichtig hydrolisiert. Die wässrige Phase wurde mit verdünnter KOH alkalisch gemacht und ausgeethert.

Die vereinigten organischen Phasen wurden über Natriumsulfat getrocknet, das Lösungsmittel entfernt und der Rückstand destilliert.

Ausbeute: 74,8 g = 53,7%
bp. = 71 - 73°C/15 mm
Lit.: analog W.A. White, H.Weingarten, J.Org. Chem. *32*, 213 (1967)

B. 5-Oxo-2,4-dimethylheptanitril

Eine Lösung aus 63 g (0,425 Mol) A und 30,3 g (0,425 Mol) Methacrylnitril werden 20 Stunden in 500 ml abs. Ethanol unter Rückfluss zum Sieden erhitzt. Anschliessend versetzt man mit 120 ml Wasser und kocht nochmals 90 min. Danach wird die Hauptmenge des Lösungsmittels entfernt, mit 300 ml Wasser versetzt und mehrmals mit Ether extrahiert. Die Etherphase wird mehrmals mit verdünnter HCl gewaschen, über Natriumsulfat getrocknet, eingeengt und im Vakuum destilliert.

Ausbeute: 32,3 g = 46,6%
bp. = 107 - 113°C/15 mm
Lit.: analog G. Storck et al., J.A.C.S. *85*, (1967)

C. 5-Hydroxy-2,4-dimethylheptanitril

Zu einer Lösung aus 5,15 g (0,136 Mol) Natriumborhydrid in 500 ml abs. Ethanol werden 32,3 g (0,211 Mol) B vorsichtig dazugetropft und anschliessend zwei Tage rühren gelassen. Die Hydrolyse erfolgt mit ca. 100 ml verdünnter HCl. Danach wird das Lösungsmittel abgezogen und der Rückstand mit Ether aufgenommen, mit Wasser gewaschen und über Natriumsulfat getrocknet. Nach Entfernung des Lösungsmittels wird der Rückstand im Vakuum destilliert.

Ausbeute: 25,5 g = 77%
bp. = 130°C/15 mm
Lit.: analog Organikum

D. 4,6-Dimethyl-7-hydroxy-3-nonanon

Zu einer Lösung aus 0,41 Mol Ethylmagnesiumbromid (aus 44,8 g Ethylbromid und 10 g Magnesiumspänen) in 600 ml abs. Ether, werden 25,5 g (0,164 Mol) C in 50 ml abs. Ether vorsichtig zugetropft und die Lösung 3 h unter Rückfluss zum Sieden erhitzt. Danach wird über Nacht mit gesättigter Ammoniumchloridlösung hydrolysiert, die Etherphase abgetrennt, die wässrige Phase mehrmals mit Ether extrahiert und die vereinigten organischen Phasen über Magnesiumsulfat getrocknet.

Das Rohprodukt wird nicht isoliert, sondern sofort weiterverarbeitet.

Aus dem Rohprodukt lässt sich nach Entfernen des Lösungsmittels (in neutralem Medium) auch Serricornin isolieren. Das Verfahren gemäss den Stufen A bis D stellt somit auch ein vorzügliches Verfahren zur Herstellung von Serricornin dar.

E. 2,6-Diethyl-3,5-dimethyl-2,3-dihydro-4H-pyran

Die Etherphase mit D wird ca. 1 Stunde über 10%iger Salzsäure gerührt, abgetrennt und die wässrige Phase mehrmals mit Ether extrahiert. Die vereinigten Etherextrakte trocknet man über einer Mischung aus Natriumsulfat und Natriumcarbonat, entfernt das Lösungsmittel und destilliert im Vakuum.

Ausbeute: 7,3 g = 26,5%

bp. = 89 - 93°C/20 mm

Das gemäss dem vorstehenden Beispiel erhaltene Anhydroserricornin ist mit natürlichem, im Sexuallockstoff des weiblichen Tabakkäfers *Lasioderma serricorne* identisch. Das natürliche Anhydroserricornin wurde durch gaschromatische Untersuchung eines Pheromonextraktes aus unbegatteten weiblichen Tabakkäfern zunächst gaschromatographisch nachgewiesen und isoliert. Hierzu diente eine Gaschromatograph Fractovap 2101 der Firma Carlo Erba mit einer Glaskapillare (WCOT belegt mit Superox als Trennphase), Länge 25 m, Durchmesser 0,2 mm. Als Trägergas diente Helium (3 ml/min); der Split betrug 1 : 20; das Temperaturprogramm wurde wie folgt eingestellt:

Temperaturprogramm: 60 bis 180°C mit 3,3°C/min.

Der Anhydroserricornin-Peak erschien nach etwa 4 Minuten; der Peak des Serricornins nach etwa 20 Minuten.

Die Struktur des Anhydroserricornins wurde durch Massenspektroskopie ermittelt und letztlich durch die unabhängige Synthese gemäss dem vorstehenden Ausführungsbeispiel bestätigt.

Im folgenden wird über Versuche zum Vergleich der Pheromon-Aktivität zwischen Serricornin und Anhydroserricornin berichtet.

*Lasioderma serricorne*, die auch das Gewebe von anderen Pflanzenarten als *Nicotiana tabacum* angreifen, wurden aus zerkleinertem Mais mit einem Zusatz von 5% getrockneter Bierhefe bei 26 ± 1 °C und 55 ± 5% relativer Luftfeuchte sowie einer Photoperiode von L 14 : D 10 gezüchtet. Männliche und weibliche Käfer wurden entweder durch äussere Erkennunngsmerkmale der Puppen oder mikroskopische Betrachtung der Geschlechtsorgane der Imagines erkannt.

Zum Vergleich der Pheromon-Aktivität von Serricornin und Anhydroserricornin wurden Versuche mit laborgezüchteten Populationen von Tabakkäfern durchgeführt, und zwar in gleichen Anzahlen von männlichen und weiblichen Tieren. 50 männliche und 50 weibliche Tabakkäfer in einem Alter von 5 bis 10 Tagen nach dem Schlupf wurden 24 Stunden in einem 2 l Glasgefäss, bedeckt mit perforierter Aluminiumfolie mit Zentralschlitz, konditioniert. An die Abdeckung der Glasgefässe wurden rechteckige Filterpapierstreifen (12 × 3 cm), imprägniert mit steigenden Mengen von Serricornin oder Anhydroserricornin, gehängt. Bald danach konnte man beobachten, dass die männlichen Käfer in Richtung auf den kleinen Bereich (Durchmesser etwa 8 mm) fliegen, der die eine oder die andere der vorgenannten Komponenten enthält. Nach der Landung wandern die Käfer in einem kreisförmigen und zickzackförmigen Muster zu der Pheromonquelle, wo sie für unterschiedliche Zeitspannen aggregieren. Die Anzahl und Dauer der Besuche und Kopulationsversuche erwiesen sich als abhängig von der vorhandenen Komponente und Dosierung (Beobachtungszeit 30 min). Ein Vergleich dieser Parameter zeigt, dass die Aktivität von Anhydroserricornin etwa 1000mal grösser als die des Serricornins ist. Innerhalb eines Bereichs von $10^{-6}$ bis $10^{-2}$ $\mu$g ist der Anstieg der Attraktionswirkung bei Anhydroserricornin beträchtlich steiler als für Serricornin. Bei einer Dosis von $10^{-1}$ $\mu$g und mehr zeigt sich eine Adaption an beide Komponenten (cf. Fig. 1). In Gegenwart von $10^{-4}$ bis zu $10^{0}$ $\mu$g Anhydroserricornin versammeln sich männliche Tabakkäfer in grosser Zahl auf beiden Seiten des behandelten Papierabschnitts und versuchen, miteinander zu kopulieren. In gleicher Menge aufgetragenes Serricornin veranlasst lediglich einige wenige Käfer zu dieser Versammlung und Kopulation.

**Patentansprüche für die Vertragsstaaten:**
BE, CH, DE, FR, LI, LU, NL, SE

1. 2,6-Diethyl-3,5-dimethyl-2,3-dihydro-4H-pyran.

2. Verfahren zur Herstellung von 2,6-Diethyl-3,5-dimethyl-2,3-dihydro-4H-pyran, dadurch gekennzeichnet, dass man 4,6-Dimethyl-7-hydroxy-3-nonanon cyclisiert.

3. Mittel zur Früherkennung, Lokalisierung und Bekämpfung von Lasioderma serricorne F., enthaltend 2,6-Diethyl-3,5-dimethyl-2,3-dihydro-4H-pyran sowie übliche feste oder flüssige Trägerstoffe, Insektizide und/oder Insektistatica.

4. Mittel nach Anspruch 3, dadurch gekennzeichnet, dass es in einer Insektenfalle enthalten ist.

5. Verwendung von 2,6-Diethyl-3,5-dimethyl-2,3-dihydro-4H-pyran zur Früherkennung, Lokalisierung und Bekämpfung von Lasioderma serricorne F.

**Patentanspruch für den Vertragsstaat:** AT

Verfahren zur Herstellung von2,6-Diethyl-3,5-dimethyl-2,3-dihydro-4H-pyran, dadurch gekennzeichnet, dass man 4,6-Dimethyl-7-hydroxy-3-nonanon cyclisiert.

**Claims for the Contracting States:**
BE, CH, DE, FR, LI, LU, NL, SE

1. 2,6-Diethyl-3,5-dimethyl-2,3-dihydro-4H-pyran.

2. Process for preparing 2,6-diethyl-3,5-dimethyl-2,3-dihydro-4H-pyran, characterized in that 4,6-dimethyl-7-hydroxy-3-nonanone is cyclized.

3. Composition for early detection, localization and combating of Lasioderma serricorne F., comprising 2,6-diethyl-3,5-dimethyl-2,3-dihydro-4H--pyran and common solid or liquid carriers, insecticides and/or insect control agents.

4. Composition according to claim 3, characterized in that it is contained in an insect trap.

5. The use of 2,6-Diethyl-3,5-dimethyl-2,3-dihydro-4H-pyran for early detection, localization and combating of Lasioderma serricorne F.

**Claim for the Contracting State:** AT

Process for preparing 2,6-diethyl-3,5-dimethyl-2,3-dihydro-4H-pyran, characterized in that 4,6-dimethyl-7-hydroxy-3-nonanone is cyclized.

**Revendications pour les Etats Contractants:**
BE, CH, DE, FR, LI, LU, NL, SE

1. 2,6-diéthyl-3,5-diméthyl-2,3-dihydro-4H-pyranne.

2. Procédé pour la préparation de 2,6-diéthyl-3,5-diméthyl-2,3-dihydro-4H-pyranne, caractérisé en ce qu'on cyclise la 4,6-diméthyl-7-hydroxy-3-nonanone.

3. Produit pour l'identification précoce, la localisation et la destruction de Lasioderma serricorne F., contenant du 2,6-diéthyl-3,5-diméthyl-2,3-dihydro-4H-pyranne, ainsi que des substances vectrices, des insecticides et/ou des insectistatiques usuels, solides ou liquides.

4. Produit selon la revendication 3, caractérisé en ce qu'il est contenu dans un piège à insectes.

5. Utilisation de 2,6-diéthyl-3,5-diméthyl-2,3-dihydro-4H-pyranne pour l'identification précoce, la localisation et la destruction de Lasioderma serricorne F.

**Revendication pour l'Etat Contractant:** AT

Procédé pour la préparation de 2,6-diéthyl-3,5-diméthyl-2,3-dihydro-4H-pyranne, caractérisé en ce qu'on cyclise la 4,6-diméthyl-7-hydroxy-3-nonanone.

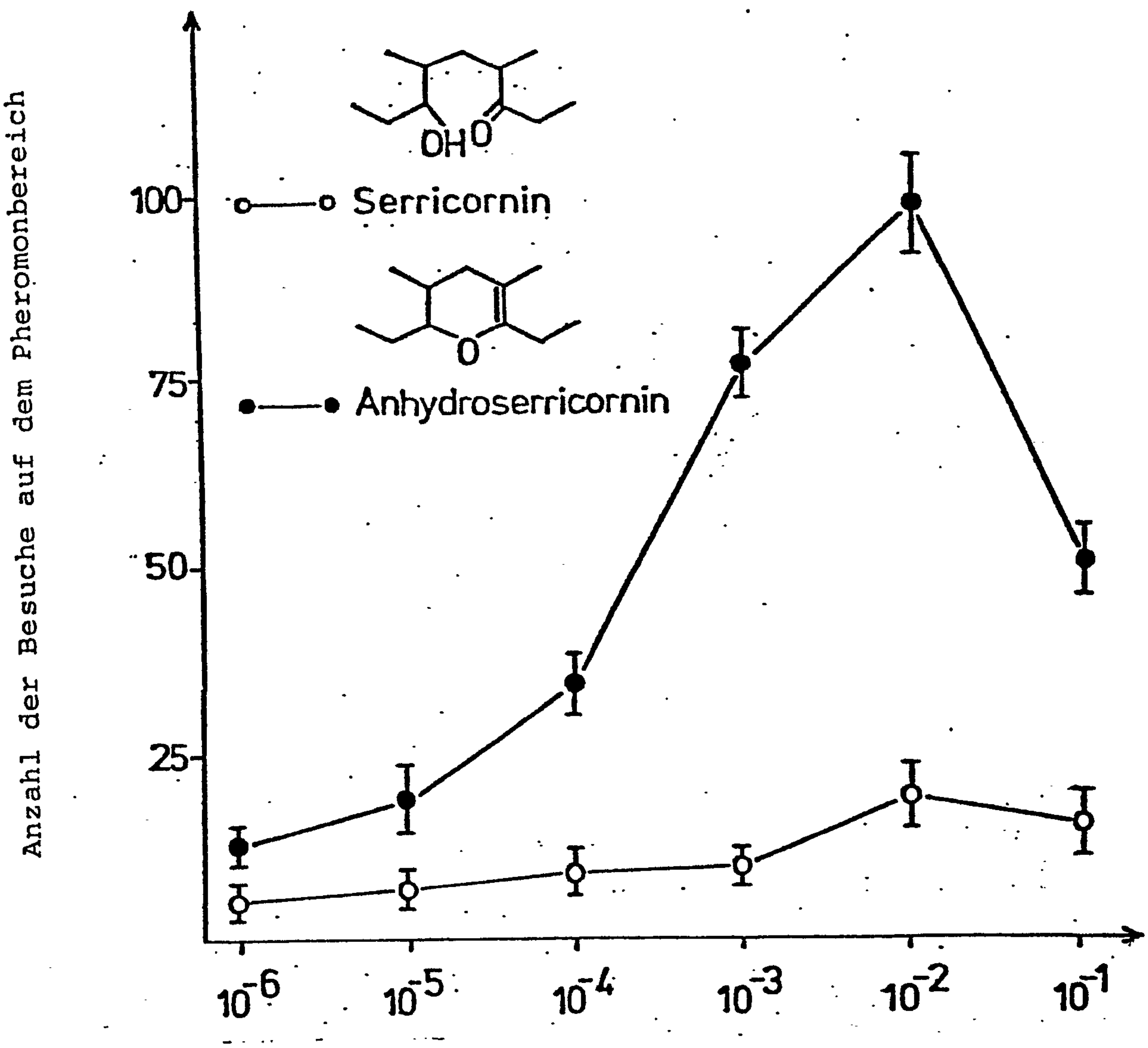
Serricornin
Anhydroserricornin
100
75
50
25
10^-6
10^-5
10^-4
10^-3
10^-2
10^-1
Anzahl der Besuche auf dem Pheromonbereich
Aufgetragene Dosis µg

Fig. 1